(19) Europäisches Patentamt
European Patent Office
Office européen des brevets

(11) **EP 4 613 327 A1**

(12) **EUROPEAN PATENT APPLICATION**

(43) Date of publication:
**10.09.2025 Bulletin 2025/37**

(21) Application number: **25161288.3**

(22) Date of filing: **03.03.2025**

(51) International Patent Classification (IPC):
**A61N 5/10** (2006.01)    **G16H 20/40** (2018.01)

(52) Cooperative Patent Classification (CPC):
**A61N 5/103; A61N 5/1047; G16H 20/40;**
A61N 5/1036; A61N 5/1038; A61N 5/1045;
A61N 2005/1041; A61N 2005/1062

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **04.03.2024 US 202418595280**

(71) Applicant: **Siemens Healthineers International AG**
**6312 Steinhausen (CH)**

(72) Inventors:
• **ARBERET, Simon**
**Princeton, 08540 (US)**
• **KUUSELA, Esa**
**02320 Espoo (FI)**
• **GHESU, Florin-Cristian**
**91083 Baiersdorf (DE)**
• **COMANICIU, Dorin**
**Princeton, 08540 (US)**
• **GAO, Riqiang**
**Plainsboro, 08536 (US)**
• **KAMEN, Ali**
**Skillman, 08558 (US)**

(74) Representative: **Mathisen & Macara LLP**
**Charta House**
**30-38 Church Street**
**Staines-upon-Thames TW18 4EP (GB)**

(54) **SYSTEMS AND METHODS FOR GENERATING RADIATION THERAPY TREATMENTS PLANS**

(57)      Provided herein are systems for planning radiotherapy treatments. Systems can include one or more processors to receive radiotherapy data associated with a set of treatments administered to a set of patients; generate a beam eye view (BEV) projection for each patient of the set of patients; and for each patient of the set of patients, provide treatment data associated with the patient and data associated with the BEV projections corresponding to the patient to a model to train the model to generate an output. The output can represent a fluence map. Systems and method for generating leaf sequences are also provided.

FIG. 2

EP 4 613 327 A1

# Description

## TECHNICAL FIELD

This application relates generally to systems and methods for generating radiation therapy treatment plans.

## BACKGROUND

Radiation therapy (referred to as radiotherapy (RT)) is a common technique used to treat certain forms of cancer. RT involves careful planning to deliver radiation to targets within the body of a patient. During treatment, a multi-leaf collimator (MLC) comprising multiple leaves can be moved relative to a patient. Between each control point at which energy is delivered, the leaves of the MLC can be positioned and repositioned during the delivery of radiation by the linear accelerator (LINAC) to form the shape of the beam at each control point. The goal of carefully planning operation of the LINAC is to deliver radiation to the tumor while minimizing the delivery of radiation to surrounding healthy tissues. As will be appreciated, programming the LINAC to position and reposition to deliver radiation to a tumor is a complex process, which in large part relies on the expertise of trained clinicians.

Recently, advanced software-based systems were developed to assist when developing complex RT plans in an effort to minimize normal tissue damage and target margins. For example, knowledge-based planning, which uses previous knowledge to guide subsequent treatment planning, has attracted wide attention recently in the context of medical imaging and treatment. However, certain aspects of the radiotherapy therapy treatment planning process cannot easily be addressed using existing knowledge-based planning techniques. For example, techniques suitable for analyzing two-dimensional images are often difficult to translate to the three-dimensional domain. As a result, conventional inverse optimization problems continue to be the standard when performing certain portions of the treatment planning process. These techniques are costly, both in terms of the time they require and the computational resources they consume.

## SUMMARY

For the aforementioned reasons, there is a need for systems and methods that can rapidly and accurately analyze scans of a patient and automate one or more aspects of the radiotherapy treatment planning process.

In an embodiment, a system can comprise one or more processors programmed to: receive radiotherapy treatment data associated with a set of treatments administered to a set of patients that were previously treated, where each treatment of the set of treatments corresponds to a patient of the set of patients; generate a beam eye view (BEV) projection for each patient of the set of patients based on treatments of the set of treatments administered to each patient of the set of patients; and for each patient of the set of patients, provide treatment data associated with the patient and data associated with the BEV projections corresponding to the patient to a model to train the model to generate an output, the output representing a fluence map.

The one or more processors programmed to generate the BEV projection for each patient of the set of patients can be programmed to: generate a set of digitally reconstructed radiographs (DRRs) based on the treatments of the set of treatments for each patient of the set of patients; and concatenate the set of DRRs for each patient to form the BEV projection for the patient.

The one or more processors programmed to generate the set of DRRs can be programmed to: generate the set of DRRs based on the treatments of the set of treatments for each patient of the set of patients and configurations of a multi-leaf collimator (MLC) involved in the treatments of the set of treatments.

The one or more processors programmed to provide the treatment data associated with each patient and the data associated with the BEV projections for each patient to the model can be programmed to: provide the treatment data associated with the patient, and the data associated with the BEV projections for each patient to the model, the treatment data associated with the patient comprising a representation of a planning target volume (PTV) or a representation of an organ at risk (OAR) for the patient.

The one or more processors programmed to provide the treatment data associated with each patient and the data associated with the BEV projections associated with each patient to the model can be programmed to: provide the treatment data associated with the patient, and the data associated with the BEV projections for each patient to the model, the treatment data associated with the patient comprising one or more leaf configurations of a multi-leaf collimator (MLC) during transmission of energy by a linear accelerator (LINAC) to the patient.

The one or more processors programmed to provide the treatment data associated with each patient and data associated with the BEV projections associated with each patient to the model to train the model to generate an output can be programmed to: compare the output to a target fluence map to determine a difference between the output and the target fluence map; and update one or more weights of the model based on the difference between the output and the target fluence map.

The one or more processors programmed to provide treatment data associated with each patient and data associated with the BEV projections associated with teach patient to the model to train the model to generate an output can be programmed to: provide the treatment data associated with the patient and the data associated with the BEV projections associated with the patient to the model to train the model to generate a first output, the first output associated with a first scale; and provide the treatment data associated with the patient and the data associated with the BEV projections associated with the patient to the model to train the model to generate the output, the output associated with a second scale.

In another embodiment, a method can comprise: receiving, by at least one processor, radiotherapy treatment data associated with a set of treatments administered to a set of patients that were previously treated, where each treatment of the set of treatments corresponds to a patient of the set of patients; generating, by the at least one processor, a beam eye view (BEV) projection for each patient of the set of patients based on treatments of the set of treatments administered to each patient of the set of patients; and for each patient of the set of patients, providing, by the at least one processor, treatment data associated with the patient and data associated with the BEV projections corresponding to the patient to a model to train the model to generate an output, the output representing a fluence map.

Generating the BEV projection for each patient of the set of patients can comprise: generating, by the at least one processor, a set of digitally reconstructed radiographs, DRRs, based on the treatments of the set of treatments for each patient of the set of patients; and concatenating, by the at least one processor, the set of DRRs for each patient to form the BEV projection for the patient.

Generating the set of DRRs can comprise: generating, by the at least one processor, the set of DRRs based on the treatments of the set of treatments for each patient of the set of patients and configurations of a multi-leaf collimator, MLC, involved in the treatments of the set of treatments.

Providing the treatment data associated with each patient and the data associated with the BEV projections associated with each patient to the model can comprise: providing, by the at least one processor, the treatment data associated with the patient and the data associated with the BEV projections for each patient to the model, the treatment data associated with the patient comprising a representation of a planning target volume, PTV, or a representation of an organ at risk, OAR, for the patient.

Providing the treatment data associated with each patient and the data associated with the BEV projections associated with each patient to the model can comprise: providing, by the at least one processor, the treatment data associated with the patient and the data associated with the BEV projections for each patient to the model, the treatment data associated with the patient comprising one or more leaf configurations of a multi-leaf collimator, MLC, during transmission of energy by a linear accelerator, LINAC, to the patient.

Providing the treatment data associated with each patient and the data associated with the BEV projections associated with each patient to the model to train the model to generate an output can comprise: comparing, by the at least one processor, the output to a target fluence map to determine a difference between the output and the target fluence map; and updating, by the at least one processor, one or more weights of the model based on the difference between the output and the target fluence map.

Providing the treatment data associated with each patient and the data associated with the BEV projections associated with each patient to the model to train the model to generate an output can comprises: providing, by the at least one processor, the treatment data associated with the patient and the data associated with the BEV projections associated with the patient to the model to train the model to generate a first output, the first output associated with a first scale; and providing, by the at least one processor, the treatment data associated with the patient and the data associated with the BEV projections associated with the patient to the model to train the model to generate the output, the output associated with a second scale.

In yet another embodiment, a non-transitory computer-readable medium stores instructions thereon that, when executed by one or more processors, cause the one or more processors to: receive radiotherapy treatment data associated with a set of treatments administered to a set of patients that were previously treated, where each treatment of the set of treatments corresponds to a patient of the set of patients; generate a beam eye view (BEV) projection for each patient of the set of patients based on treatments of the set of treatments administered to each patient of the set of patients; and for each patient of the set of patients, provide treatment data associated with the patient and data associated with the BEV projections corresponding to the patient to a model to train the model to generate an output, the output representing a fluence map.

By virtue of the implementation of the techniques described in association with the above-noted systems and methods, the time needed for treatment planning in the context of IMRT and VMAT therapies can be significantly reduced by replacing costly inverse planning optimization processes with a direct single path approach using a model (e.g., a machine learning-based model as described herein). With particular focus on VMAT therapies, the fluence maps that are predicted by virtue of the above-noted techniques account for the mechanical constraints of the linear accelerator and reduce or eliminate the need for conventional iterative processes when generating fluence maps. In the context of IMRT therapies, while conventional methods focus on predicting the fluence maps for each angle separately, the techniques described herein jointly predict fluence maps for each angle simultaneously. This has the advantage of improving the global optimization of treatment planning as each angle specified by a treatment plan must be determined relative to the others to avoid overshooting or undershooting certain areas of the body. Again, with respect to VMAT therapies, it is even more important to predict the fluence maps jointly because the resulting leaf sequence must be continuous across control points to avoid mechanical errors that can arise out of sudden motion of the LINAC, collision with leaves of the MLC, etc.

## BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.

**FIG. 1** illustrates a diagram of a system for generating radiation therapy treatment plans, according to an embodiment.

**FIG. 2** illustrates a flow diagram of a process for generating fluence maps, according to an embodiment.

**FIG. 3A** illustrates an example of geometric properties of multiple beams represented in a BEV image, according to an embodiment.

**FIG. 3B** illustrates a model used to predict the fluence map according to an embodiment.

**FIG. 3C** illustrates a diagram of a multi-scale approach for training a model, according to an embodiment.

**FIG. 4** illustrates a flow diagram of a process for generating leaf sequences, according to an embodiment.

**FIG. 5A** illustrates an example flow diagram of a model that can be trained to receive data associated with a fluence map as input and provide data associated with a leaf sequence as output, according to an embodiment.

**FIG. 5B** illustrates examples of a target fluence and possible fluences that can be predicted using a model, according to an embodiment.

**FIG. 5C** illustrates a model that receives as input fluence map data and multi-leaf collimator data and outputs a leaf sequence, according to an embodiment.

**FIG. 5D** illustrates a visual depiction of input-output pair mappings for supervised training of one or more models, according to an embodiment.

**FIG. 5E** illustrates a set of chained models and an example visual depiction of progression of the chained model through a search space, according to an embodiment.

**FIG. 6** illustrates is flow diagram of a process for generating fluence maps and leaf sequences, according to an embodiment.

**FIG. 7** illustrates an example flow diagram of an implementation of two models in sequence, according to an embodiment.

## DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments can be used and/or other changes can be made without departing from the scope of the present disclosure. The illustrative embodiments described in the detailed description are not meant to be limiting of the subject matter presented.

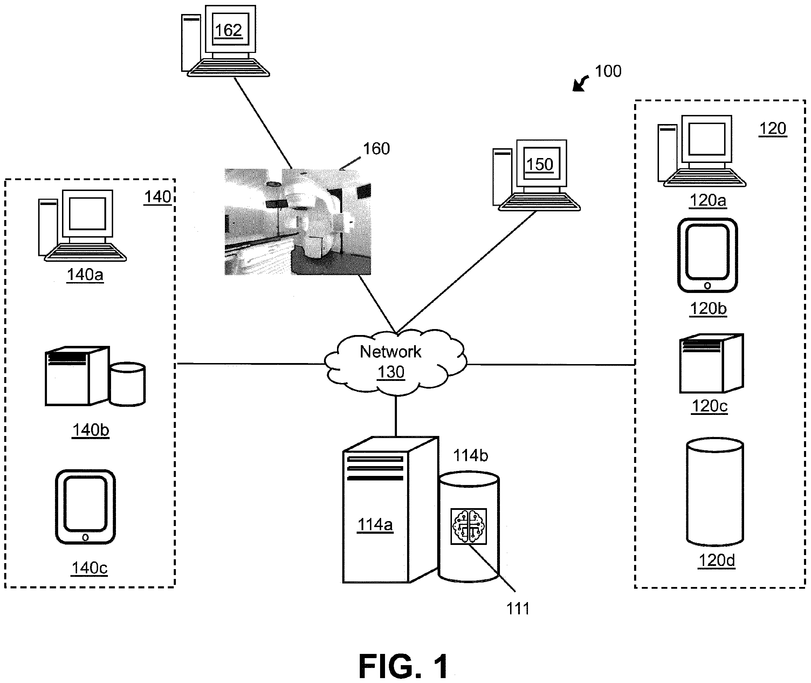

**FIG. 1** illustrates components of a system **100** for planning and delivering radiation therapy, according to an embodiment. The system **100** can include an analytics server **114a,** system database **114b,** a model **111** (e.g., a machine learning-based model as described herein), electronic data sources **120a-d** (collectively electronic data sources **120**), end-user devices **140a-c** (collectively end-user devices **140**), an administrator computing device **150,** a medical device **160,** and medical device computer(s) **162.** Various components depicted in **FIG. 1** can belong to a radiotherapy clinic at which patients can receive radiotherapy treatment, in some cases via one or more radiotherapy machines located within the clinic (e.g., medical device **160**). The system **100** is not confined to the components described herein and can include additional or other components, not shown for brevity, which are to be considered within the scope of the embodiments described herein.

The above-mentioned components can be connected to each other through a network **130.** Examples of the network **130** can include, but are not limited to, private or public local-area-networks (LAN), wireless LAN (WLAN) networks, metropolitan area networks (MAN), wide-area networks (WAN), and the Internet. The network **130** can include wired and/or wireless communications according to one or more standards and/or via one or more transport mediums. The communication over the network **130** can be performed in accordance with various communication protocols such as Transmission Control Protocol and Internet Protocol (TCP/IP), User Datagram Protocol (UDP), and IEEE communication protocols. In one example, the network **130** can include wireless communications according to Bluetooth specification sets or another standard or proprietary wireless communication protocol. In another example, the network **130** can also include communications over a cellular network, including, e.g., a GSM (Global System for Mobile Communications), CDMA (Code Division Multiple Access), and EDGE (Enhanced Data for Global Evolution) network.

The analytics server **114a** can generate and display an electronic platform configured to use a model **111** (including artificial intelligence and/or machine learning models) for receiving patient information and outputting the results of execution of the model **111.** The electronic platform can include graphical user interfaces (GUI) displayed on one or more electronic data sources **120,** the end-user devices **140,** the medical device **160,** and/or the administrator computing device **150.** An example of the electronic platform generated and hosted by the analytics server **114a** can be a web-based application or a website configured to be displayed on different electronic devices, such as mobile devices, tablets, personal computers, and the like.

The information displayed by the electronic platform can include, for example, input elements to receive data associated with a patient being treated, synchronize one or more sensors, and display results of predictions produced by the model **111.** For instance, the analytics server **114a** can execute the model **111** (e.g., machine learning models trained to generate fluence maps, leaf sequences, etc., as described herein for a patient being treated via the medical device **160**). The analytics server **114a** can then display the results for a clinician and/or directly revise one or more operational attributes of the medical device **160.**

The analytics server **114a** can be any computing device comprising a processor and non-transitory machine-readable storage capable of executing the various tasks and processes described herein. The analytics server **114a** can employ various processors such as central processing units (CPU) and graphics processing unit (GPU), among others. Non-limiting examples of such computing devices can include workstation computers, laptop computers, server computers, and the like. While the system **100** includes a single analytics server **114a,** the analytics server **114a** can include any number of computing devices operating in a distributed computing environment, such as a cloud environment.

The electronic data sources **120** can represent various electronic data sources that contain, retrieve, and/or access data associated with a medical device **160,** such as operational information associated with previously performed radiotherapy treatments (e.g., electronic log files or electronic configuration files), data associated with previously monitored patients (e.g., CT scans, tumor locations, deformation information, and/or the like) or participants in a study to train the AI models discussed herein. For instance, the analytics server **114a** can use the clinic computer **120a,** medical professional device **120b,** server **120c** (associated with a clinician and/or a clinic), and database **120d** (associated with the clinician and/or the clinic) to retrieve/receive data associated with the medical device **160.** The analytics server **114a** can retrieve the data from the end-user devices **120,** generate a training dataset, and train the AI models **111.** The analytics server **114a** can execute various algorithms to translate raw data received/retrieved from the electronic data sources **120** into machine-readable objects that can be stored and processed by other analytical processes as described herein.

End-user devices **140** can be any computing device comprising a processor and a non-transitory machine-readable storage medium capable of performing the various tasks and processes described herein. Non-limiting examples of an end-user device **140** can be a workstation computer, laptop computer, tablet computer, or server computer. In operation, various users can use end-user devices **140** to access the GUI operationally managed by the analytics server **114a** or otherwise the results of the execution of the model **111.** Specifically, the end-user devices **140** can include clinic computer **140a,** clinic server **140b,** and a medical professional device **140c.** Even though referred to herein as "end-user" devices, these devices can not always be operated by end-users. For instance, the clinic server **140b** can not be directly used by an end user. However, the results stored on the clinic server **140b** can be used to populate various GUIs accessed by an end user via the medical professional device **140c.** In some embodiments, the end-user device **140** can be associated with one or more clinicians that are associated with one or more treatment plans (e.g., involved in preparing the one or more treatment plans) for patients).

The administrator computing device **150** can represent a computing device operated by a system administrator. The administrator computing device **150** can be configured to display radiotherapy treatment attributes generated by the analytics server **114a** (e.g., various analytic metrics determined during training of one or more machine learning models and/or systems); monitor various models **111** utilized by the analytics server **114a,** electronic data sources **120,** and/or end-user devices **140;** review feedback; and/or facilitate training or retraining (calibration) of the model **111** that are maintained by the analytics server **114a.**

In some embodiments, the medical device **160** can be a diagnostic imaging device or a treatment delivery device. For example, the medical device 160 can include one or more computed tomography (CT) scanners, linear accelerators (LINACs) having a multi-leaf collimator (referred to herein as a collimator for ease of description) that consists of multiple small lead leaves that can be individually moved to shape the radiation beam and deliver the dose to the tumor while minimizing the dose to surrounding healthy tissues, or other similar devices configured to transmit energy toward targeted tissue (referred to as planning target volumes) associated with a patient and, in some cases, measure the energy transferred toward the targeted tissue. The medical device **160** can also include one or more sensors configured to monitor the patient being treated. That is, the medical device **160** and/or the analytics server **114a** can be communicating with various sensors that can monitor a patient's external biological signals. Non-limiting examples of the sensors can include 3D surfacing mechanisms and optical (or other) sensors configured to monitor the patient's movements (e.g., how the patient is moving and/or breathing.

The model **111** can be stored in the system database **114b.** The model **111** can be trained using data received/retrieved from the electronic data sources **120** and can be executed using data received from the end-user devices, the medical device **160,** and/or the sensor **163**. In some embodiments, the model **111** can reside within a data repository local or specific to a clinic. In various embodiments, the model **111** can use one or more deep learning engines to develop a treatment plan for a patient using radiation therapy. For instance, the analytics server **114a** can transmit patient attributes from the sensor **163** and execute the model **111** accordingly. The analytics server **114a** can then display the results on one or more end-user devices **140.** In some embodiments, the analytics server **114a** can change one or more configurations of the medical device 160 based on the results predicted by the model **111.**

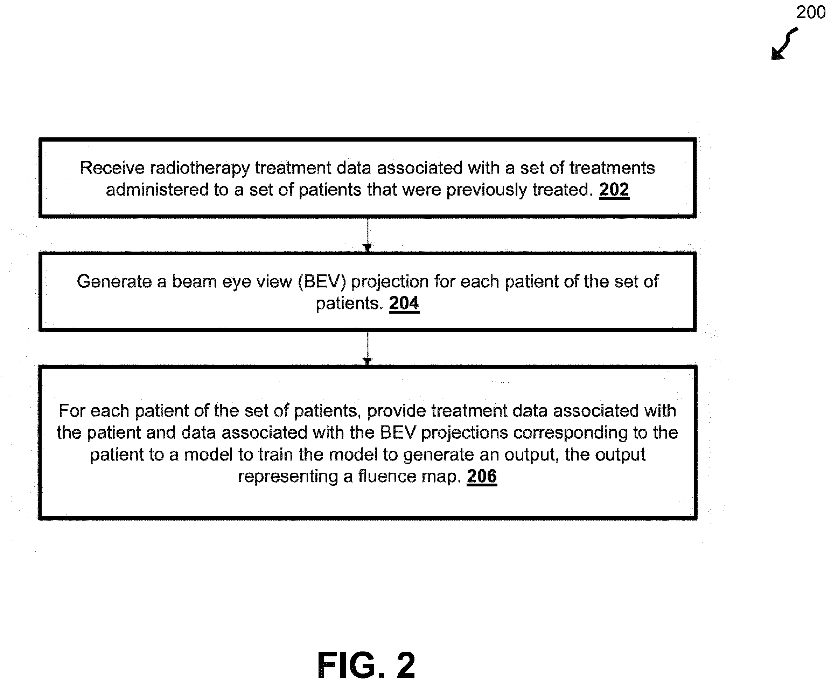

Referring to **FIG. 2,** illustrated is flow diagram of a process **200** for generating fluence maps, in accordance with an embodiment. The process **200** includes operations **202-206**. However, other embodiments can include additional or alternative operations or can omit one or more operations altogether. The process **200** is described as being executed by an analytics server, which can be the same as, or similar to, the analytics server **114a** described in **FIG. 1.** However, one or more steps of the process **200** can be executed by any number of computing devices operating in the distributed computing system described in **FIG. 1.** For instance, one or more computing devices can locally perform part or all of the steps described in **FIG. 2.**

At operation **202,** the analytics server receives radiotherapy treatment data associated with a set of treatments administered to a set of patients that were previously treated. For example, the analytics server can receive radiotherapy treatment data associated with a set of patients, where each of the patients was treated using a LINAC configured to delivery energy in accordance with an intensity-modulated radiation therapy (IMRT) plan or a volumetric modulated arc therapy (VMAT) plan. In some embodiments, the radiotherapy treatment data associated with the set of patients can include (e.g., represent) a set of LINAC configurations (e.g., LINAC gantry positions, multi-leaf collimator (MLC) configurations, and corresponding monitor unit (MU) values). For example, the radiotherapy treatment data associated with the set of patients can include a set of LINAC configurations across a plurality of points in time that are associated with treatment of a patient. In this example, the radiotherapy treatment data can represent one or more leaf sequences as described herein. In some embodiments, the radiotherapy treatment data can represent one or more of a PTV or an OAR as described herein.

In some embodiments, the radiotherapy treatment data associated with the set of patients can include (e.g., represent) a set of computed tomography (CT) scans. In examples the radiotherapy treatment data associated with the set of patients can include (e.g., represent) a set of magnetic resonance imaging (MRI) scans. In some examples, the radiotherapy treatment data associated with the set of patients can include (e.g., represent) a set of ultrasound scans.

At operation **204,** the analytics server generates a beam eye view (BEV) projection for each patient of the set of patients. For example, the analytics server can generate at least one BEV projection for each patient based on the radiotherapy treatment data associated with a set of treatments. In examples, the analytics server can generate the BEV projection based on subsets of the radiotherapy treatment data corresponding to each patient of the set of patients. In some embodiments, the analytics server generates a plurality of BEV projections for each patient of the set of patients. As will be understood, the BEV projections can correspond to treatment of each patient during periods of time associated with radiotherapy treatment assigned to the patient.

In some embodiments, the analytics server generates the BEV projection for each patient based on the analytics server generating a set of digitally reconstructed radiographs (DRRs). For example, the analytics server can generate a set of DRRs based on the radiotherapy treatment therapy data and one or more configurations of the LINAC involved in the performance of the treatment therapy. In this example, the analytics server can generate the set of DRRs, where one or more DRRs correspond to patients of the one or more patients whose treatment is represented by the radiotherapy treatment data. The one or more configurations of the LINAC can represent positions of the gantry of the LINAC relative to the patient and/or the configuration of the leaves of the MLC supported by the LINAC. The analytics server can then combine each DRR corresponding to a treatment session for a given patient to form the BEV projection.

In one example, the analytics server can combine each DRR for a patient by concatenating each DRR associated with a treatment (e.g., a treatment session). The concatenated DRRs can represent the radiation delivered to the patient from a specific point of view. For example, the concatenated DRRs can be used to determine a BEV of a plane extending through a portion of a planning target volume (PTV). In this example, the delivery of radiation associated with multiple control points can be represented in a single image. The resulting BEV image can represent the delivery of radiation both to a PTV and to one or more organs in a vicinity of the PTV. These organs in the vicinity of the PTV are referred to as organs at risk (OARs).

At operation 206, for each patient of the set of patients, the analytics server provides treatment data associated with the patient and data associated with the BEV projections corresponding to the patient to a model to train the model to generate an output, the output representing a fluence map. For example, the analytics server can provide the treatment data associated with the patient and data associated with the BEV projections corresponding to the patient to the model, where the treatment data represents one or more of: a representation of a PTV associated with the patient and/or a representation of one or more OARs of the patient. In examples, the PTV and the one or more OARs can be represented in association with (e.g., included in) a three-dimensional representation of a portion of the body of the patient. In examples, the analytics server can determine the representation of the portion of the body based on one or more scans (e.g., CT scans, MRI scans, and/or the like) involving the patient.

In some embodiments, the analytics server can provide the treatment data associated with the patient and the data associated with the BEV projections corresponding to the patient to a model to train the model, where the treatment data associated with the patient represents one or more leaf configurations of an MLC supported by a LINAC. For example, the one or more leaf configurations can be leaf configurations corresponding to each beam of a plurality of beams generated by the LINAC during treatment of the patient. In examples, the one or more leaf configurations can be further associated with one or more MU values representing the amount of radiation delivered while the LINAC supported the MLC in a given leaf configuration.

In some embodiments, the analytics server can provide the treatment data associated with the patient and the data associated with the BEV projections corresponding to the patient to the model where the model comprises one or more encoders and one or more decoders. For example, where the model is associated with a U-net architecture, the model can comprise one or more encoders associated with a contracting path and one or more corresponding decoders associated with an expanding path, the one or more encoders and one or more decoders being associated with one or more levels. In this example, the analytics server can provide the treatment data associated with the patient and the data associated with the BEV projections corresponding to the patient to the model by providing individual BEV images and corresponding treatment data (e.g., corresponding LINAC configurations and MU values) to a first encoder at a first level of the model. The first encoder can be configured to receive the individual BEV image and provide two outputs: a first output which is provided by the first encoder to a second encoder along the contracting path, and a second output which is provided to a first decoder which corresponds to the first encoder, the first decoder associated with the expanding path. Depending on the number of layers of the model, the process can be repeated for corresponding encoders and decoders at each successive layer of the model. In some embodiments, at the last (or n-th) layer of the model, the output of the encoder at the n-th layer is provided as the input of the decoder at the n-th layer. The decoder then provides an output to the decoder associated with the layer above the n-th layer, which continues for each successive layer until reaching the first decoder.

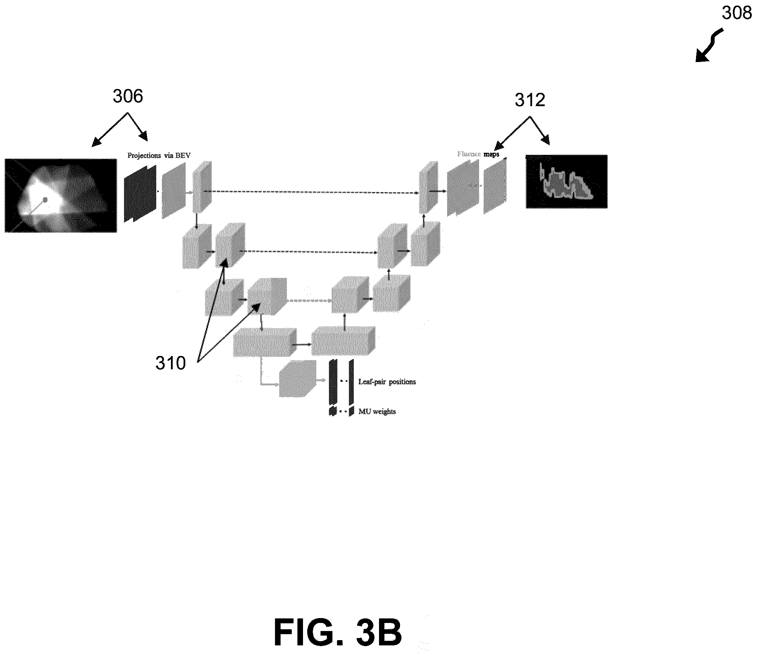

In some embodiments, the outputs of each encoder can be provided to one or more convolution layers that are associated with the encoder before being provided to the next encoder along the contracting path of the model or, in the case of the n-th encoder, the n-th decoder along the expanding path of the model. Additionally, or alternatively, the output of each encoder can be provided to a max pooling layer (e.g., after passing through the one or more convolution layers) before being provided to the next encoder along the contracting path of the model. In some embodiments, the output of each decoder along the expanding path of the model can be provided to an up-convolution layer. Additionally, or alternatively, the output of each decoder can be provided to one or more convolution layers (e.g., after passing through the up-convolution layer). In some embodiments, the last decoder of the model can provide as an output the one or more fluence maps. For example, the last decoder of the model can provide as an output the one or more fluence maps where the one or more fluence maps correspond to the one or more BEV images that are provided as input to the model. An example model implementing a U-net architecture is described with respect to the illustration of FIG. 3B.

While the present disclosure is discussed with respect to a model that can be, in examples, associated with a U-net architecture, one of ordinary skill will appreciate that other models (e.g., a V-net model, a convolutional neural network (CNN), and/or the like) can be involved in one or more operations of process 200.

In some embodiments, the analytics server can update one or more weights of the model based on the output of the model. For example, where the model receives as input treatment data associated with the patient and the data associated with the BEV projections corresponding to the patient, the model can provide as an output data associated with fluence maps. In this example, the analytics server can iteratively provide single instances of BEV projections and corresponding treatment data associated with a given patient to cause the model to output a corresponding fluence map. In this example, the analytics server can then compare the fluence map to a target fluence map to determine a difference between the fluence map output by the model and the target fluence map. In some embodiments, the target fluence map is associated with (e.g., included in or stored in association with) the treatment data associated with the patient.

In some embodiments, the analytics server can update one or more weights of the model based on the analytics server comparing the fluence map output by the model at each iteration to a target fluence map, where the target fluence map represents one or more dose-height variation (DHV) profiles. For example, the analytics server can compare the DHV profiles represented by the fluence map output from the model to the DHV profiles of the target fluence map. The analytics server can then determine a difference between the DHV profile of the output of the model to the DHV profile of the target fluence map and update the one or more weights of the model as described above.

In some embodiments, the analytics server can update one or more weights associated with the model based on the difference between the fluence map output by the model and the target fluence map. For example, where the difference satisfies a threshold amount, the analytics server can update one or more weights associated with the model to cause a successive input of the same data to the model to more closely approximate the target fluence map. In this example, the threshold amount can represent a variation in one or more corresponding pixels, a variation in one or more intensity values of corresponding pixels that meets or exceeds a predetermined intensity value, and/or the like. The analytics server can repeat this aspect of operation 206 until the model converges.

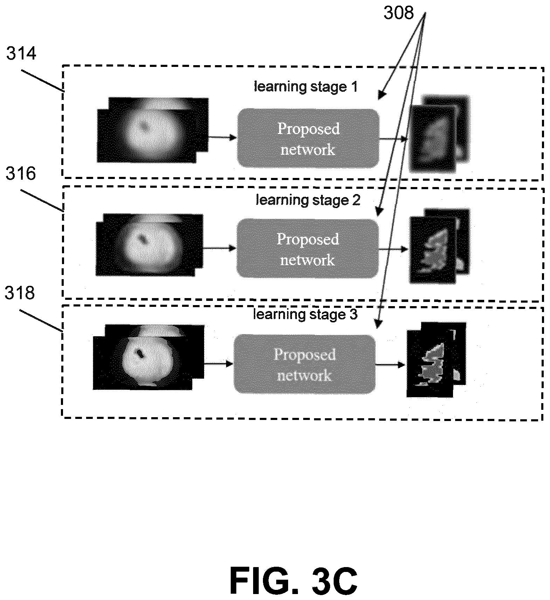

In some embodiments, the analytics server can provide the treatment data associated with the patient and the data associated with the BEV projections corresponding to the patient in one or more stages. For example, the analytics server can provide the treatment data associated with the patient and the data associated with the BEV projections corresponding to the patient in one or more stages. In this example, the one or more stages can correspond to one or more scales at which the model is trained. An illustration of training at multiple stages is discussed with respect to FIG. 3C. For example, at a first scale, the analytics server can provide the treatment data associated with the patient and the data associated with the BEV projections corresponding to the patient where the treatment data is restricted to instances where the MLC is transmitting radiation at a first predetermined set of beam angles (e.g., angles measured between the beams formed by the MLC as supported by the LINAC relative to an axis extending along the height of the patient relative to an isocenter). Additionally, or alternatively, at the first scale, the analytics server can provide the treatment data associated with the patient and the data associated with the BEV projections corresponding to the patient where the treatment data is restricted to instances where the MLC is transmitting radiation while the leaves of the MLC are confined to a second predetermined range. In this example at a second scale, the analytics server can provide the treatment data associated with the patient and the data associated with the BEV projections corresponding to the patient where the treatment data is restricted to instances where the MLC is transmitting radiation at a second predetermined set of beam angles. Here, the second predetermined set of beam angles can be greater than the first predetermined set of beam angles (e.g., the beam angles can be spaced closer together within the second predetermined set of beam angles when compared to the first predetermined set of beam angles) Additionally, or alternatively, at the second scale, the analytics server can provide the treatment data associated with the patient and the data associated with the BEV projections corresponding to the patient where the treatment data is restricted to instances where the MLC is transmitting radiation while the leaves of the MLC are confined to a second predetermined range. In some embodiments, the one or more beam angles associated with the first scale can be included as a subset of beam angles in the second scale.

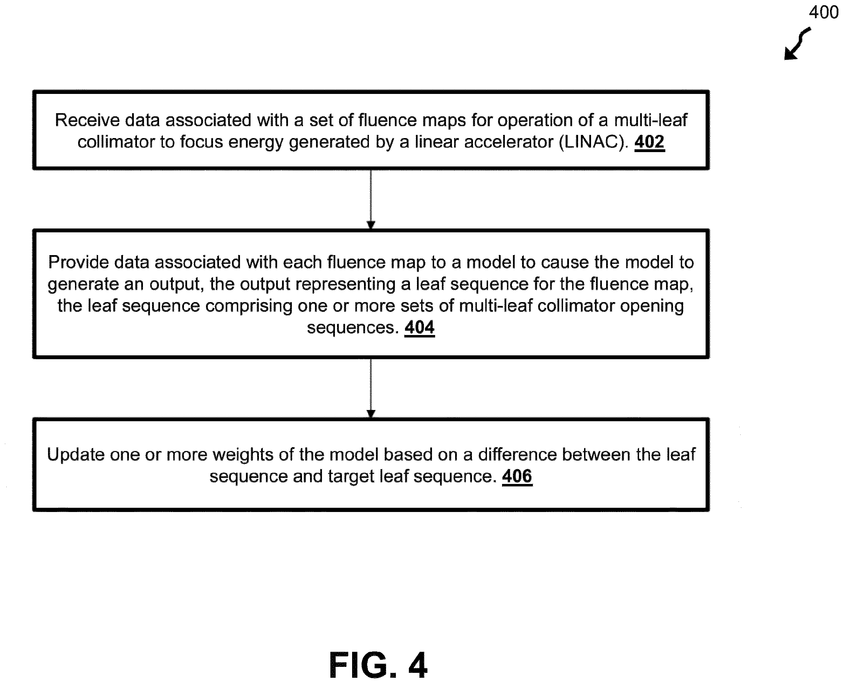

While training at different scales, given a number of training iterations $i$, $(0 < i < N)$, both input BEV projections and output fluence maps as well as an arc dimension (the dimension of K representing the control points described herein) are gradually refined across multiple stages from a first stage to a second, third, and so on. At each successive stage, the scale described above can be reduced (e.g., downscaled) to include increasingly more details when training at subsequent stages, thereby gradually refining the network features to high-frequency components. Figure 4 illustrates an example of how the network can be trained in multiple training stages, starting form low-resolutions inputs and outputs pairs (e.g., a first predetermined scale), and progressively introducing the high frequency components (e.g., a second predetermined scale, a third predetermined scale, and so on).

By virtue of implementing the disclosed techniques, the need for initial fluence map iterations based on inverse planning techniques prior to leaf sequencing can be eliminated. Additionally, the need for repetitive confirmation that a given fluence map corresponds to a leaf sequence that results in an acceptable dose calculation can also be reduced or eliminated entirely. This is because the fluence maps that are predicted by models as described herein can be trained on fluence maps represented by earlier RT plans that were optimized during the treatment planning process. Further, the fluence maps generated based on the techniques described herein have a higher probability of satisfying the operational constraints of the LINAC and supported MLC, and by extension have a higher probability of being performable by the LINAC.

Further, in the case of VMAT, certain techniques for optimizing VMAT treatment plans can rely on a multi-

resolution approach where a given arc is first divided in large sectors containing, e.g., 16 control points, and then the sectors are split in two in each subsequent multi-resolution stage, starting from, e.g., 16 control points sectors, to 8, 4 and finally 2. The goal of this multi-resolution approach is to avoid converging into local minima which can occur where there is a discrepancy between the optimal fluence map and what the LINAC can realize given its mechanical constraints. As the modes described herein are trained based on fluence maps which are the result of optimized VMAT treatment plans, the target fluence maps already consider the machine constraints. As a result, few (if any) additional steps need to be performed to confirm the fluence maps described herein are optimal.

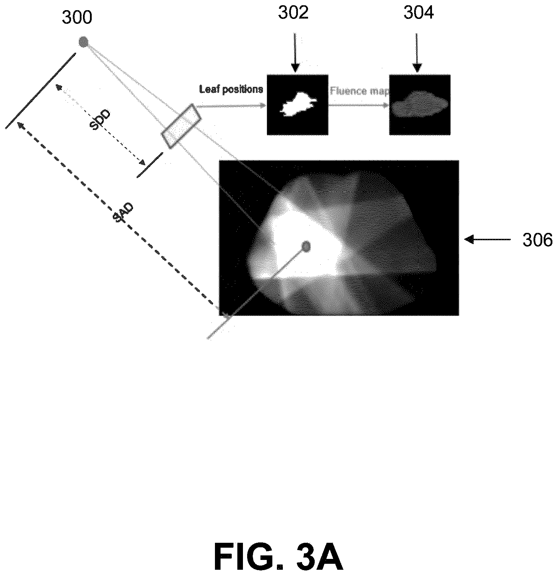

Referring to **FIG. 3A,** illustrated is an example of geometric properties of multiple beams represented in a BEV image, according to an embodiment. More specifically, **FIG. 3A** illustrates multiple beams generated by an MLC supported by a LINAC, the beams are directed toward an isocenter associated with the PTV of a patient. In this example, individual DRRs are concatenated to form the illustrated BEV image. In some embodiments, the analytics server can predict a fluence map based on the BEV image generated in accordance with the process **200,** described above. In some embodiments, the prediction can be made for each of (or at least a portion of) the control points associated with different gantry angles of the LINAC as described herein.

As illustrated, the projections represented by the BEV image are obtained based on one or more DRRs. As described above, a DRR can refer to an image generated based on one or more images from a simulated projection of radiation through an anatomy of a patient (referred to as "projections"). The projections can then be illustrated along one or more planes extending through the anatomy of the patient. In some embodiments, the analytics server can create the DRRs by digitally simulating the radiation attenuation properties of the tissues of the patient based on the medical images (e.g., computed tomography (CT) and/or magnetic resonance imaging (MRI) data) generated of the patient during delivery of radiation to the patient (e.g., toward the PTV). In some embodiments, the analytics server can simulate different MUs for the different beam angles as represented by the BEV image.

In some embodiments, the analytics server can use a location **300** of a radiation source and a distance from the location **300** to the MLC (referred to as a source-to-detector distance or SDD) to predict how the radiation interacts with (e.g., reaches) tissue of the patient. In this projection, the analytics server can also consider the leaf positions **302** (e.g., representing a shape of the MLC opening when forming a given beam) and fluence map **304** along with a source axis distance (SAD distance). The analytics server can then predict the BEV image **306,** which is specific to the location **300**. In some embodiments, the BEV image **306** is specific to a beam angle associated with a position of the radiation source (e.g., location **300**) relative to the patient. In some embodiments, the analytics server can iteratively predict other BEV images by iteratively moving the location **300** (e.g., based on a defined increment or based on different control points). In some embodiments, the analytics server can concatenate different DRRs (associated with different beams formed by the MLC) as a multi-channel input (e.g., 2D) for a model configured to receive data associated with the BEV and provide as an output a fluence map as described herein.

In the above example, using the DRR, the analytics server can simulate interactions between an anatomy of a patient and radiation delivered to the patient and the analytics server can represent the simulated interactions via radiographic images without exposing the patient to actual radiation. Moreover, the analytics server can generate DRRs at various angles based on the available treatment data, thereby eliminating the need to retrieve the same or similar data from previously implemented treatments. As a result, the analytics server can generate (e.g., simulate) the treatment data used to train the models described herein without being constrained to datasets including only previously-generated data associated with a given patient.

Referring to **FIGS. 3B and 3C,** illustrated is a non-limiting example of a model 308 used to predict the fluence map as described with respect to process **200.** Though **FIG. 3B** depicts a model based on a U-net architecture, the techniques described herein are not limited to such models and one skilled in the art will readily appreciate that the techniques described herein can be applied to any suitable model (e.g., any suitable neural network) such as, for example, a conditional variational autoencoder (CVAE) and/or the like.

As illustrated in **FIG. 3B,** BEV images **306** are generated by the analytics server (e.g., based on projections as captured in the DRRs that are used to generate the BEV images) and can be provided as input to the model **308**. In one example, the analytics server can provide the BEV images **306** as an input to the model **308** where the BEV images **306** are based on simulated projections. The analytics server can then cause the model **308** to determine correspondences between fluence maps **312** and BEV images **306**. In some embodiments, the analytics server can train the model **308** to identify a mapping between the BEV images **306** to a fluence map **312** that corresponds to the projection. For instance, the model **308** can include various convolutional layers **310** that the analytics server trains to encode and then decode the BEV images **306** based on the MU weights and position of the MLC (e.g., leaf pair position or MLC opening as discussed with respect to **FIG. 3A**) corresponding to each BEV image **306**.

In some embodiments, in addition to providing the BEV images **306**, the analytics server can provide data associated with different organs (e.g., contours of organs), contours containing PTVs and OARs, via an additional input channel associated with inputs to the model **308.** For example, during training, the analytics server can provide data associated with different organs, contours containing PTVs and OARs, etc., via additional input channels associated with inputs to the model **308.** In examples, at inference, the analytics server can provide data associated with different organs via an additional input channel associated with inputs to the model **308.**

In some embodiments, after the analytics server trains the model **308,** the analytics server can configure the model **308** to output fluence maps **312,** which can represent a prediction of all K beams $\hat{y} = \{\hat{y}_i\}_{i=1}^{K}$.

For example, during training, the analytics server can update weights of the model **306** based on various loss functions to improve the accuracy of the model **306.** In one embodiment, the loss function for training is defined as:

$$L = \sum_{i=1}^{K} Dist(y_i, \hat{y}_i)$$

where the $y_i$ is the referenced fluence map of i-th beam, $Dist(\cdot,\cdot)$ is the distance between $y_i$ and $\hat{y}_i$. In some embodiments, the $Dist(\cdot,\cdot)$ can represent a distance function that measures a distance between a predicted value and a ground truth value, the distance being determined based on a calculation of one or more of an L1 norm (sometimes referred to as an L1 distance), an L2 norm (sometimes referred to as an L2 distance), a mean squared error (MSE), a structural similarity index measure (SSIM), and/or combinations thereof. Additionally, or alternatively, the loss function can be represented as a loss function applicable to a generative adversarial network (GANs), including Wasserstein GANs (WGANs) or a loss function that is computed within a distinct space, such as a dose space. For example, a loss function can be denoted as

$$L = Dist(target\_dose, dosec(\hat{y}))$$

where dosec(.) represents a differentiable function that computes a dose distribution from the output fluence maps **312,** and target_dose = dosec(y) is a target dose distribution.

When the model **308** is executed based on VMAT treatments, the K beams can correspond to K control points (e.g., a discretization of the arc in K consecutive angles). In those embodiments, in addition to predicting the fluence maps **312,** an additional branch (sometimes referred to as a detection head) can be added to the model **308** to output a leaf-sequencing prediction (e.g., sets of right leaves, left leaves, and MU weights for each angle of the K angles). This leaf sequencing can be used as an initialization for a leaf-sequencing module.

In some embodiments, during training or validation, the analytics server can implement various assessments protocols (e.g., assessing the accuracy of prediction). For example, in addition to validating the target fluence map (using suitable thresholds as described herein), the dose domain can also be validated (e.g., by leveraging DVH profiles).

Referring to **FIG. 3C,** illustrated is a diagram of a multi-scale approach for training a model as described herein. More specifically, the analytics server can implement a multi-scale approach during training to increase robustness during training. A schematic representation of this technique is depicted in **FIG. 3C.**

In some embodiments, during training iterations, both the BEV images provided as input to the model and the fluence maps provided as output from the model (as well as the arc dimensions in some embodiments) can be gradually refined from a downscaled starting version to include increasingly more details, thereby gradually refining the network features to high-frequency components. **FIG. 3C** illustrates how the model **308** can be trained in multiple training stages, starting form low-resolutions inputs and outputs pairs on the initial learning state **314,** and progressively introducing the high frequency components (e.g., the second learning stage **316**) until the model **308** is fully trained (e.g., last learning stage **318**).

**FIG. 4** illustrates a flow diagram of a process **400** for generating leaf sequences, according to an embodiment. The process **400** includes operations **402-406.** However, other embodiments can include additional or alternative operations or can omit one or more operations altogether. The process **400** is described as being executed by an analytics server, which can be the same as, or similar to, the analytics server **114a** described in **FIG. 1.** However, one or more steps of the process **400** can be executed by any number of computing devices operating in the distributed computing system described in **FIG. 1.** For instance, one or more computing devices can locally perform part or all of the steps described in **FIG. 4.**

At operation **402,** the analytics server receives data associated with a set of fluence maps for operation of an MLC to focus energy generated by a LINAC. For example, the analytics server can receive the data associated with the set of fluence maps for operation of an MLC based on treatment of one or more patients. In an example, the analytics server can receive the set of fluence maps based on generation of treatment plans for patients. In this example, the patients can be involved in intensity-modulated radiation therapy (IMRT) or volumetric modulated arc therapy (VMAT) treatments. In some embodiments, the data associated with the set of fluence maps can be further associated with MUs. For example, for each fluence map represented by the set of fluence maps, a corresponding MU representing the radiation delivered at one or more points in connection with the fluence map can be included in (e.g., associated with) the data associated with the set of fluence maps.

In some embodiments, the analytics server receives the data associated with the set of fluence maps based on generation of a sample fluence map training set. For example, the analytics server can receive the data associated with the set of fluence maps based on a computing device (e.g., the analytics server or another computing device) generating a sample fluence map training set. In this example, the sample fluence training set can include one or more randomly-generated fluence maps. In examples, the sample fluence training set can include one or more fluence maps associated with the treatment of the one or more patients (discussed above). In some embodiments, the analytics server can receive data associated with the fluence maps based on the treatment of the one or more patients and the analytics server can generate the sample fluence map training set. For example, the analytics server can receive the data associated with the fluence maps based on the treatment of the one or more patients and the analytics server can add one or more randomly-generated fluence maps. In this way, the analytics server can augment the sample fluence training set by including fluence maps generated by clinicians and fluence maps that are randomly-generated.

At operation **404,** the analytics server provides data associated with each fluence map to a model to cause the model to generate an output, the output representing a leaf sequence for the fluence map, the leaf sequence comprising one or more sets of MLC opening sequences. For example, the analytics server can provide the data associated with each fluence map to a model to cause the model to generate an output during training of the model. In some embodiments, the analytics server provides the data associated with each fluence map to a model to cause the model to generate an output, where the model includes a generative transformer model. The generative transformer model can then provide the output. In each of these examples, the output includes a leaf sequence. For example, the output can include a set of leaf sequences, each leaf sequence of the set of leaf sequences representing the motion of one or more leaves of an MLC targeted to deliver radiation to tissue of a patient based on a fluence map. In some embodiments, the input to the model (e.g., a given fluence map) is associated with the output of the model (e.g., a given leaf sequence).

In some embodiments, the output includes data associated with one or more MUs. For example, the output can be associated with one or more MUs corresponding to each leaf sequence. In this example, the MUs can be associated with delivering radiation while the MLC is in a given configuration and positioned relative to the patient by the LINAC. In this way, the model can output data that is configured to cause a LINAC to control operation of a MLC in accordance with the provided fluence map.

At operation **406,** the analytics server updates one or more weights of the model based on a difference between the leaf sequence and target leaf sequence. For example, the analytics server can update the one or more weights of the model when training the model to map the fluence maps provided as input to the model to leaf sequences. In some embodiments, the analytics server trains the model based on the analytics server comparing the output of the model (e.g., the output leaf sequence) to a target leaf sequence. In this example, the target leaf sequence can correspond to a given fluence map that the analytics server provides as input to the model.

In some embodiments, the analytics server can update one or more weights associated with the model based on the difference between the fluence map output by the model and the target fluence map. For example, as noted above, the analytics server can compare the leaf sequence that is output by the model to a target leaf sequence. In this example, the analytics can determine the difference based on the comparison of the output leaf sequence and the target leaf sequence. In some embodiments, where the analytics server determines that the difference satisfies a threshold amount, the analytics server can update one or more weights associated with the model to cause a successive input of the same data to the model to more closely approximate the target fluence map. In this example, the threshold amount can represent a variation in one or more corresponding pixels, a variation in one or more intensity values of corresponding pixels that meets or exceeds a predetermined intensity value, and/or the like. The analytics server can repeat this aspect of operation **406** until the model converges.

In some embodiments, the analytics server can train the model to map the fluence maps provided as input to the model to leaf sequences, where the leaf sequences are included in a subset of leaf sequences from among a set of leaf sequences. For example, the analytics server trains the model to map the fluence maps provided as input to the model to a subset of leaf sequences that are possible (e.g., that are associated with leaf motion sequences and positions that a LINAC can perform). In this example, the subset of leaf sequences that are possible can be associated with a set of all possible leaf sequences.

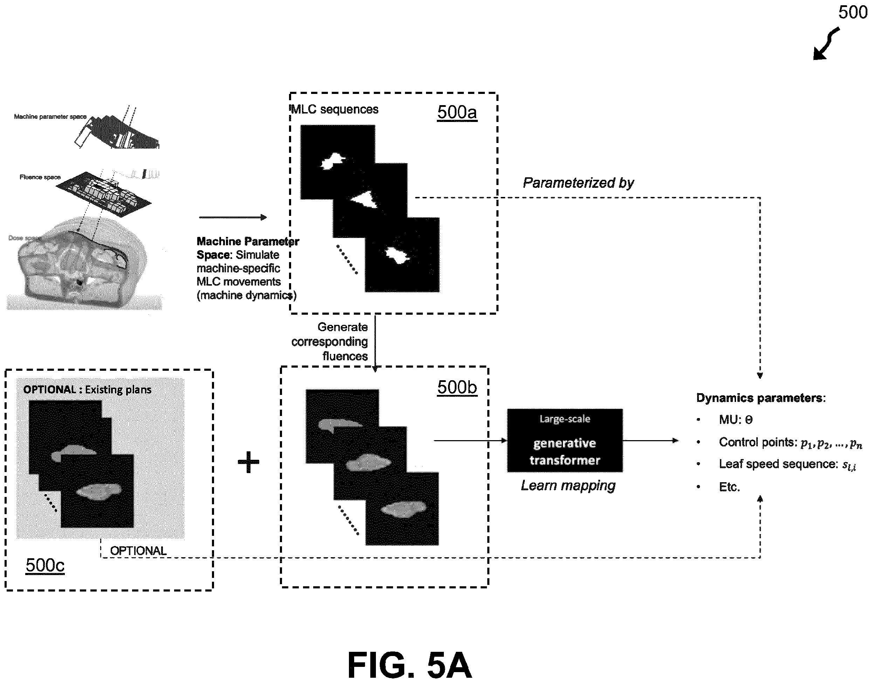

Referring to **FIG. 5A,** illustrated is an example flow diagram of a model **500** that can be trained to receive data associated with a fluence map as input and provide data associated with a leaf sequence as output. In some embodiments, the model **500** can be the same as, or similar to, the models described with respect to process **400 of FIG. 4.** As illustrated, a model **500** (e.g., a machine learning-based and/or deep learning-based model as described herein) that learns to map from fluences to the parameters describing aspects of leaves of an MLC during operation (including MU levels (0), leaf speed sequence $s_{l,i}$ indicating the position of a given leaf $l$ at control point i, the control points $p_1, p_2, ..., p_n$, etc.). One skilled in the art will readily appreciate that, in addition to the models described herein, the techniques described can be applied to any suitable model (e.g., any suitable neural network).

In some embodiments, the model **500** can learn to map from fluence to dynamics of MLC without the need to ingest data associated with treatment plans. It will be understood that in some embodiments, existing or historical data associated with treatment plans can be provided to the model **500** to augment its performance. By eliminating the dependencies on existing or historical data associated with treatment plans, the analytics server can configure the model **500** to predict MLC sequences for "new" plans (e.g., plans that are not based on prior treatments of patients) without the need to retrieve extensive historical data, which can be time-consuming and resource intensive. Moreover, because the model **500** is not dependent upon historical and previous radiotherapy treatment data associated with treatment plans, the model **500** can predict new radiotherapy treatment plans that can better optimize performance at inference as compared to models trained only using radiotherapy treatment data associated with treatment plans that were involved in actual treatments.

In some embodiments, the model **500** can be a controllable parametric model of the LINAC, capturing the dynamics of the leaves of the MLC, dose rate settings, and/or the like. Using the model 500 (that has been trained based on one or more of the techniques described herein), large numbers of fluence maps can be predicted, thereby establishing a correspondence between dynamics parameters associated with treatment plans and fluences.

The model **500** can ingest MLC sequences **500a** that have been simulated and generated by the analytics server (or another server) and corresponding fluence maps **500b**. The MLC sequences **500a** can be simulated using machine-specific constraints. In some embodiments, the model **500** can map the MLC sequences to their corresponding fluence maps, such that model **500** can be trained to determine how to generate MLC sequences from a fluence map, e.g., via various parameters (e.g., using dynamic parameters, such as MU values, controls points, and leaf speed sequences). For example, the model **500** can map the MLC sequences to their corresponding fluence maps, where the model **500** includes a generative transformer. In some embodiments, the model **500** can optionally ingest existing or historical treatment plans (e.g., existing or historical fluence maps) to improve the training of the model **500.**

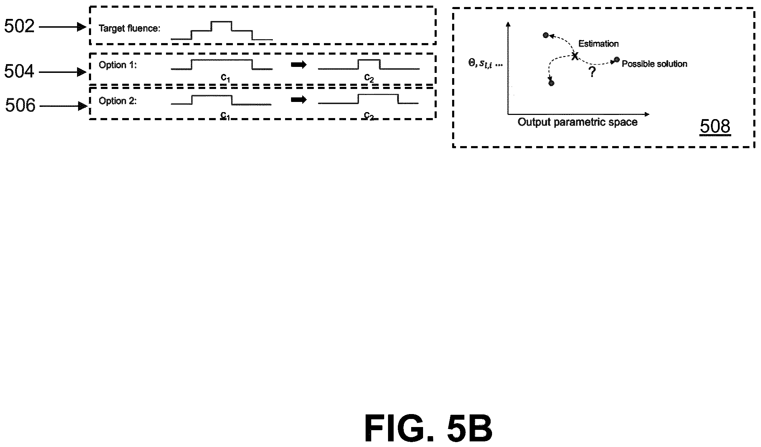

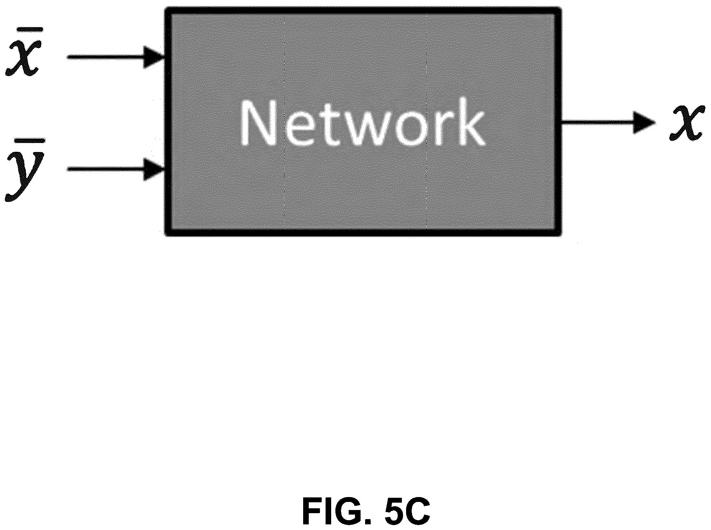

Referring to **FIG. 5B** and **5C,** illustrated are examples of a target fluence **502** and possible fluences that can be predicted using a model **500.** In some embodiments, the model **500** can learn from mapping schemes that are "one-to-many." As a result, the model **500** can predict different MLC data for the same fluence map, as illustrated in **FIG. 5B.** For example, the model **500** can predict the fluence maps **504** and **506** in order to achieve the target fluence **502.**

In some embodiments, the analytics server can first define the input/output (I/O) framework, where the inputs consist of all fluence maps of the arc, and the output encompasses the dynamic parameters across the entire arc, such as for VMAT. In examples, instead of solely estimating the MLC data from fluence maps, the analytics server can estimate the MLC data from both the fluence map $(\bar{x})$ and MLC $(\bar{y})$ data. For instance, as depicted in **FIG. 5C,** the network (e.g., the model **500)** can ingest both the fluence map $(\bar{x})$ and MLC $(\bar{y})$ data and output a single data point (e.g., x). This approach can be conceptualized as a trained projection operator mapping the optimization variables (fluence maps, MLC) to the feasible set of machine capabilities.

In some embodiments, $x$ can denote the leaf-sequence (e.g., it contains the leaf positions as well as the MU weights of each control point). In some embodiments, $y$ can denote the fluence maps. Therefore, C = {($x$, $y$) $|\phi(x) = y, x \in X$} where $X$ is the set of constraints on the MLC dynamics. The constraints, as used herein, can be machine specific and can be described mathematically. In operation, the constraints can correspond to maximum/minimum leaf speeds (e.g., 2-4 cm/s), maximum/minimum dynamic leaf gaps, maximum/minimum leaf span, maximum/minimum leaf position, and/or the like.

As a result of providing the input leaf sequences in addition to the target fluence maps, the operations of the model **500** can be simplified. More specifically, instead of searching within a broad search space and optimizing the optimal solution in the entire search space, the model **500** can be guided towards a close-by solution in the optimization space. Therefore, the model **500** can generate outputs using less time and/or computing resources as would conventionally be required.

As discussed herein, the model **500** can identify various different equivalent solutions to a single problem set. Therefore, in some embodiments, training the model **500** with supervised learning approaches based on a general search space can result in a network prediction that will reduce (e.g., try to minimize) the average error with respects to all the solutions. As the set of equivalent optimal solutions may not be convex in general (as shown, e.g., in graph **508** depicted in **FIG. 5B),** the model **500** can not predict an optimal solution. However, the solution can be revised based on various machine MLC constraints.

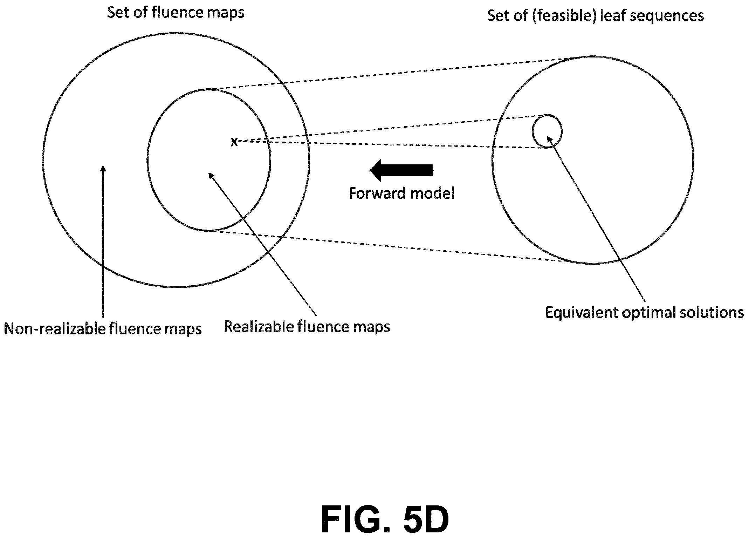

Referring to **FIG. 5D,** illustrated is a visual depiction of input-output pair mappings for supervised training of one or more models. In some embodiments, to train the model **500** in a supervised manner (e.g., to predict leaf sequences based on fluence maps and initial leaf sequences), the analytics server can create a dataset of input-output pairs as depicted herein. In examples, the input-output pairs can represent a set of fluence maps (e.g., all possible fluence maps that can be generated by one or more of the models described herein) and a set of leaf sequences (e.g., all feasible leaf sequences that can be generated by one or more of the models described herein). As discussed here, feasible leaf sequences include leaf sequences that satisfy the operation constraints of a LINAC.

In some embodiments, the set of leaf sequences can be mapped to a subset of fluence maps included in the set of fluence maps. For example, the analytics server can map the set of leaf sequences to a subset of fluence maps included in the set of fluence maps. In this example, the analytics server can map the set of leaf sequences using a forward model to the subset of fluence maps. In this way, the analytics server can identify a set of realizable fluence maps and, in cases where the analytics server receives a fluence map that does not satisfy the mapping to the set of feasible leaf sequences, can forgo generation of a leaf sequence using the fluence map.

In some embodiments, yet another set of leaf sequences can be mapped to a subset of fluence maps. For example, the analytics server can map a set of leaf sequences associated with optimal leaf sequences (e.g., leaf sequences that optimize operation of the LINAC) to a subset of fluence maps (e.g., one or more fluence maps) from among the set of realizable fluence maps. In this example, the analytics server can again map the set of leaf sequences using a forward model to the subset of fluence maps.

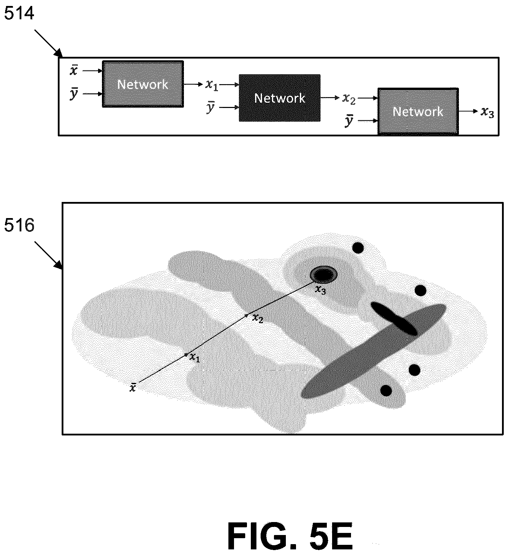

Referring to **FIG. 5E,** illustrated is a set of chained models and an example visual depiction of progression of the chained model through a search space. As a result of training the model **500** using the methods and systems discussed herein, even if the initial leaf-sequence predicted by model **500** is far from the optimal leaf sequence, the model **500** can be "chained" in order to refine the leaf-sequence in multiple steps, as depicted in **FIG. 5E** (graph **514** and **516**). Chaining the leaf-sequences can improve or stabilize the solution predicted by the model **500.**

In some embodiments, chaining of the leaf-sequences can be used to find the minimum of $\|\varphi(x) - \overline{y}\|^2$. In some embodiments, each cascade can be predicting the optimum solution of $\|\varphi(x) - \overline{y}\|^2$ in the neighboring of the current solution $x_k$, e.g., the minimum of

$$\lambda\|x - x_k\|^2 + \|\varphi(x) - \overline{y}\|^2.$$

As depicted, instead of training the model **500** to predict the final results ($X_3$), the model **500** can be trained to iteratively revise the results, such that the model **500** achieves the final results. For instance, the model **500** can receive the MLC data and fluence map and predict an initial result ($X_1$), then revise the result, such that it is closer to the optimized result (e.g., local maximum or minimum within the search space). Specifically, the model **500** identifies ($X_2$) and then ($X_3$). This iterative revision of results is also visually depicted within the graph **516** where the darker portions indicate convergence upon a result within the search space.

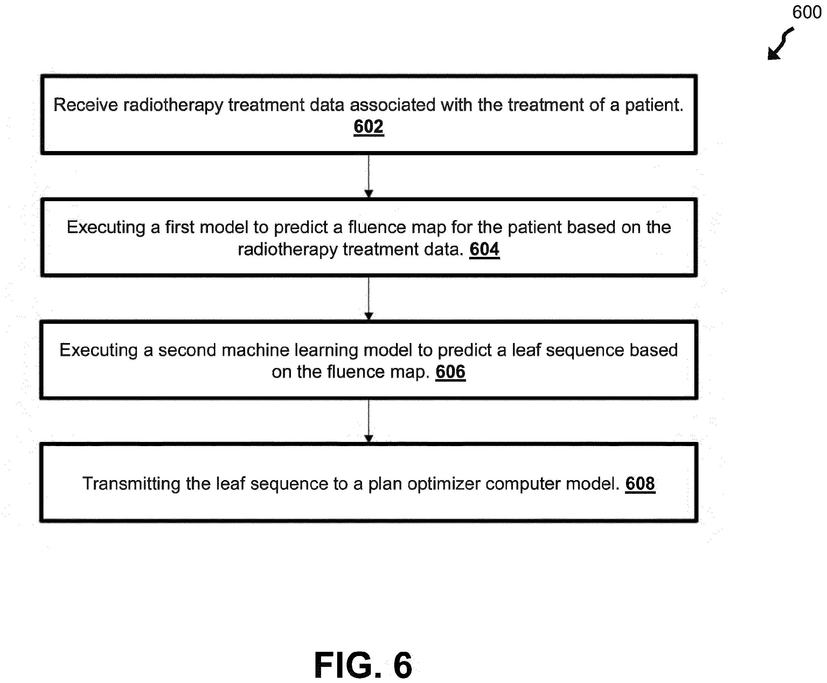

Referring to **FIG. 6,** illustrated is a flow diagram of a process **600** for generating fluence maps and leaf sequences, according to an embodiment. The process **600** includes operations **602-608**. However, other embodiments can include additional or alternative operations or can omit one or more operations altogether. The process **600** is described as being executed by an analytics server, which can be the same as, or similar to, the analytics server **114a** described in **FIG. 1.** However, one or more steps of the process **600** can be executed by any number of computing devices operating in the distributed computing system described in **FIG. 1.** For instance, one or more computing devices can locally perform part or all of the steps described in **FIG. 6.**

At operation **602,** the analytics server receives radiotherapy treatment data associated with the treatment of a patient. For example, the analytics server can receive the radiotherapy treatment data associated with the treatment of the patient, where the treatment of the patient involves IMRT or VMAT therapy. In examples, the radiotherapy treatment data associated with the treatment of the patient can include representations of one or more of: an anatomy of the patient (e.g., a CT scan, an MRI scan, and/or the like), a PTV of the patient, one or more OARs of the patient, etc.

At operation **604,** the analytics server executes a first model. For example, the analytics server can execute the first model, where the first model is the same as, or similar to, the model described with respect to process **200** of **FIG. 2.** In some embodiments, the analytics server can execute the first model to predict a fluence map for the patient. For example, the analytics server can provide the radiotherapy treatment data to the model to cause the model to generate an output. In this example, the output can include one or more fluence maps corresponding to BEV images generated by the analytics server as described herein. As described above, the analytics server can train the first model based on BEV images associated with of a set of previously-treated patients. In some embodiments, the analytics server can generate the BEV images based on the analytics server generating DRRs based on the radiotherapy treatment data and the analytics server concatenating the set of DRRs to form the BEV images.

At operation **606,** the analytics server executes a second model. For example, the analytics server can execute the second model, where the second model is the same as, or similar to, the model described with respect to process **400** of **FIG. 4.** In some embodiments, the analytics server can execute the second model to predict a leaf sequence based on a fluence map for the patient. For example, the analytics server can execute the first model to predict a fluence map for the patient and the analytics server can provide the fluence map output by the first model as input to the second model. In this example, the output can include a leaf sequence corresponding to the fluence map generated by the first model as described herein.

At operation **608,** the analytics server transmits the leaf sequence to a plan optimizer computer model. For example, the analytics server transmits the leaf sequence to a plan optimizer computer model to cause the plan optimizer computer model to output a treatment plan. In this example, the treatment plan can include data that represent one or more MLC positions when supported by a LINAC and one or more corresponding MU values that, in combination, cause the LINAC to deliver energy in accordance with the fluence map provided as input to the second model. In some embodiments, the analytics server also transmits data associated with the PTV, the OARs, and/or one or more DVHs.

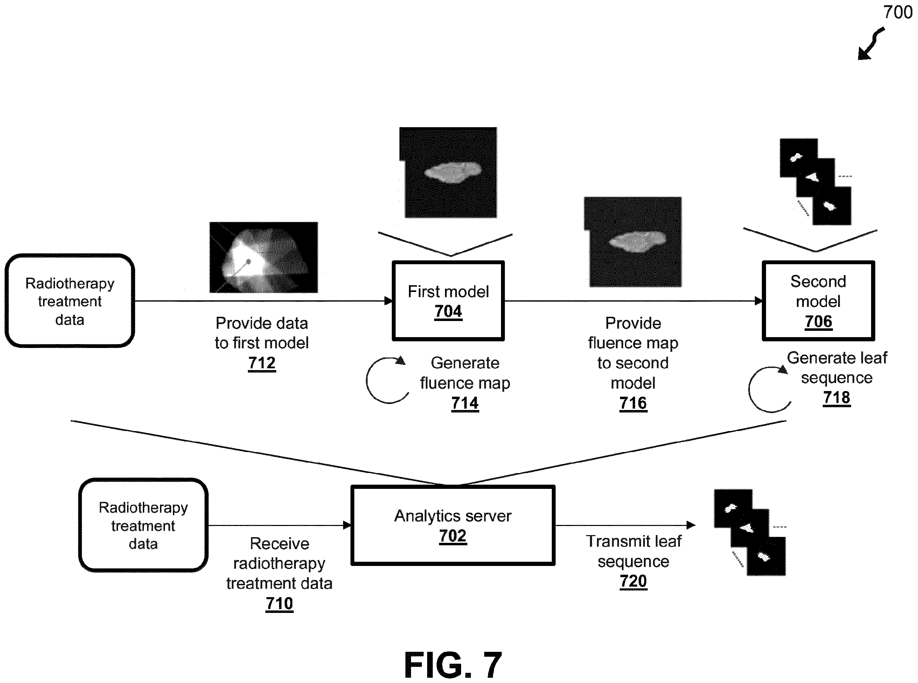

**FIG. 7** illustrates an example flow diagram of an implementation **700** of two models in sequence, according to an embodiment. The implementation **700** includes operations **710-720.** However, other embodiments can include additional or alternative operations or can omit one or more operations altogether. The implementation **700** is described as being executed by an analytics server, which can be the same as, or similar to, the analytics server **114a** described in **FIG. 1.** However, one or more steps of the implementation **700** can be executed by any number of computing devices operating in the distributed computing system described in **FIG. 1.**

At operation **710,** the analytics server **702** receives radiotherapy treatment data. For example, the analytics server **702** can receive radiotherapy treatment data associated with the treatment of a patient involving IMRT or VMAT therapies. In some embodiments, the radiotherapy treatment data represents one or more of: a PTV for the patient, one or more OARs for the patient, a scan of an anatomy of the patient, and/or the like.

At operation **712,** the analytics server **702** provides data to the first model **704.** For example, the analytics server **702** can generate one or more BEV images based on the radiotherapy treatment data. In this example, the analytics server **702** can provide data associated with the one or more BEV images to the first model **704** to cause the first model **704** to generate an output. In examples, the first model **704** is the same as, or similar to, the models described with respect to process **200** of **FIG. 2.** In some embodiments, the analytics server **702** can generate the one or more BEV images based on the radiotherapy treatment data.

At operation **714** the analytics server **702** causes the first model **704** to generate a fluence map. For example, the analytics server **702** can cause the first model **704** to generate the fluence map based on the analytics server **702** providing the data (e.g., the data associated with the one or more BEV images) to the first model.

At operation **716,** the analytics server **702** provides the fluence map to a second model **706.** For example, the analytics server **702** can provide data associated with the one or more fluence maps generated by the first model **704** to the second model **706** to cause the second model **706** to generate an output. In examples, the second model **706** is the same as, or similar to, the models described with respect to process **400** of **FIG. 4.**

At operation **718,** the analytics server **702** causes the second model **706** to generate a leaf sequence. For example, the analytics server **702** can cause the second model **706** to generate the leaf sequence based on the analytics server **702** causing the first model **704** to generate the fluence map and the analytics server **702** providing the fluence map to the second model.

At operation **720,** the analytics server **702** transmits the leaf sequence. For example, the analytics server **702** can transmit the leaf sequence to cause a LINAC to operate in accordance with the leaf sequence. In examples, the analytics server **702** can transmit data associated with the leaf sequence, the data configured to cause the LINAC to operate in accordance with the leaf sequence.

The various illustrative logical blocks, modules, circuits, and algorithm steps described in connection with the embodiments disclosed herein can be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans can implement the described functionality in varying ways for each particular application, but such implementation decisions should not be interpreted as causing a departure from the scope of this disclosure or the claims.

Embodiments implemented in computer software can be implemented in software, firmware, middleware, microcode, hardware description languages, or any combination thereof. A code segment or machine-executable instructions can represent a procedure, a function, a subprogram, a program, a routine, a subroutine, a module, a software package, a class, or any combination of instructions, data structures, or program statements. A code segment can be coupled to another code segment or a hardware circuit by passing and/or receiving information, data, arguments, parameters, or memory contents. Information, arguments, parameters, data, etc., can be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions can be stored as one or more instructions or code on a non-transitory computer-readable or processor-readable storage medium. The steps of a method or algorithm disclosed herein can be embodied in a processor-executable software module, which can reside on a computer-readable or processor-readable storage medium. A non-transitory computer-readable or processor-readable media includes both computer storage media and tangible storage media that facilitate transfer of a computer program from one place to another. A non-transitory processor-readable storage media can be any available media that can be accessed by a computer. By way of example, and not limitation, such non-transitory processor-readable media can comprise RAM, ROM, EEPROM, CD-ROM or other optical disk storage, magnetic disk storage or other magnetic storage devices, or any other tangible storage medium that can be used to store desired program code in the form of instructions or data structures and that can be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm can reside as one or any combination or set of codes and/or instructions on a non-transitory processor-readable medium and/or computer-readable medium, which can be incorporated into a computer program product.

The following clauses are part of the description, and are not claims. The Applicant reserves the right to claim this subject-matter in this application or in any divisional application filed in the future.

1. A non-transitory computer-readable medium storing instructions thereon that, when executed by one or more processors, cause the one or more processors to: receive radiotherapy treatment data associated with a set of treatments administered to a set of patients that were previously treated, where each treatment of the set of treatments corresponds to a patient of the set of patients; generate a beam eye view (BEV) projection for each patient of the set of patients based on treatments of the set of treatments administered to each patient of the set of patients; and for each patient of the set of patients, provide treatment data associated with the patient and data associated with the BEV projections corresponding to the patient to a model to train the model to generate an output, the output representing a fluence map.

2. The non-transitory computer-readable medium of clause 1, wherein the instructions that cause the one or more processors to generate the BEV projection for each patient of the set of patients cause the one or more processors to: generate a set of digitally reconstructed radiographs (DRRs) based on the treatments of the set of treatments for each patient of the set of patients; and concatenate the set of DRRs for each patient to form the BEV projection for the patient.

3. The non-transitory computer-readable medium of clause 2, wherein the instructions that cause the one or more processors to generate the set of DRRs cause the one or more processors to: generate the set of DRRs based on the treatments of the set of treatments for each patient of the set of patients and configurations of a multi-leaf collimator (MLC) involved in the treatments of the set of treatments.

4. The non-transitory computer-readable medium of any one of clauses 1 to 3, wherein the instructions that cause the one or more processors to provide the treatment data associated with the patient and the data associated with the BEV projections for each patient to the model cause the one or more processors to: provide the treatment data associated with the patient, and the data associated with the BEV projections for each patient to the model, the treatment data associated with the patient comprising a representation of a planning target volume (PTV) or a representation of an organ at risk (OAR) for the patient.

5. The non-transitory computer-readable medium of any one of clauses 1 to 4, wherein the instructions that cause the one or more processors to provide the treatment data associated with the patient and the data associated with the BEV projections for each patient to the model cause the one or more processors to: provide the treatment data associated with the patient, and the data associated with the BEV projections for each patient to the model, the treatment data associated with the patient comprising one or more leaf configurations of a multi-leaf collimator (MLC) during transmission of energy by a linear accelerator (LINAC) to the patient.

6. The non-transitory computer-readable medium of any one of clauses 1 to 5, wherein the instructions that cause the one or more processors to provide treatment data associated with the patient and data associated with the BEV projections associated with the patient to a model to train the model to generate an output cause the one or more processors to: compare the output to a target fluence map to determine a difference between the output and the target fluence map; and update one or more weights of the model based on the difference between the output and the target fluence map.

7. A system comprising: one or more processors to: receive data associated with a set of fluence maps associated with operation of a multi-leaf collimator to focus energy generated by a linear accelerator (LINAC); and provide data associated with each fluence map to a model to cause the model to generate an output, the output representing a leaf sequence for the fluence map, the leaf sequence comprising one or more sets of multi-leaf collimator opening sequences.

8. The system of clause 7, wherein the one or more processors programmed to provide the data associated with each fluence map to the model to cause the model to generate the output are programmed to: train the model to map input fluence maps to subsets of leaf sequences from among a set of possible leaf sequences.

9. The system of clause 8, wherein the one or more processors programmed to train the model to map input fluence maps to subsets of leaf sequences from among a set of possible leaf sequences are programmed to: map the input fluence maps to the subsets of leaf sequences from among a set of possible leaf sequences, where the subsets of leaf sequences are associated with one or more operational parameters of the LINAC.

10. The system of any one of clauses 7 to 9, wherein the one or more processors are further programmed to: compare the output to a target leaf sequence corresponding to each fluence map; determine a difference between the leaf sequence and the target leaf sequence; and update one or more weights of the model based on the difference between the leaf sequence and the target leaf sequence.

11. The system of any one of clauses 7 to 10, wherein the one or more processors programmed to receive the data associated with the set of fluence maps are programmed to: receive the set of fluence maps based on generation of treatment plans for patients involved in intensity-modulated radiation therapy (IMRT) or volumetric modulated arc therapy (VMAT) treatment.

12. The system of any one of clauses 7 to 10, wherein the one or more processors programmed to receive the data associated with the set of fluence maps are programmed to: receive the set of fluence maps based on generation of a sample fluence map training set, the sample fluence map training set comprising one or more randomly-generated fluence maps.

13. The system of any one of clauses 7 to 10, wherein the one or more processors programmed to receive the data associated with the set of fluence maps are programmed to: receive the set of fluence maps based on generation of a sample fluence map training set and generation of treatment plans for patients involved in intensity-modulated radiation therapy (IMRT) or volumetric modulated arc therapy (VMAT) treatment.

14. The system of any one of clauses 7 to 13, wherein the one or more processors that provide the data associated with each fluence map to the model to cause the model to generate the output are programmed to: provide the data associated with each fluence map to a generative transformer model to cause the generative transformer model to generate the output.

15. The system of any one of clauses 7 to 14, wherein the one or more processors that provide the data associated with each fluence map to the model to cause the model to generate the output are programmed to: provide the data associated with each fluence map to the model to cause the model to generate the output, the output representing a leaf sequence for the fluence map and at least one monitor unit (MU) value, the leaf sequence comprising one or more sets of multi-leaf collimator opening sequences corresponding to an MU value from among the at least one MU value.

16. A method comprising: receiving, by at least one processor, data associated with a set of fluence maps associated with operation of a multi-leaf collimator to focus energy generated by a linear accelerator (LINAC); and providing, by the at least one processor, data associated with each fluence map to a model to cause the model to generate an output, the output representing a leaf sequence for the fluence map, the leaf sequence comprising one or more sets of multi-leaf collimator opening sequences.

17. The method of clause 16, wherein the one or more processors programmed to provide the data associated with each fluence map to the model to cause the model to generate the output are programmed to: train the model to map input fluence maps to subsets of leaf sequences from among a set of possible leaf sequences.

18. The method of clause 17, wherein the one or more processors programmed to train the model to map input fluence maps to subsets of leaf sequences from among a set of possible leaf sequences are programmed to: map the input fluence maps to the subsets of leaf sequences from among a set of possible leaf sequences, where the subsets of leaf sequences are associated with one or more operational parameters of the LINAC.

19. The method of any one of clauses 16 to 18, wherein the one or more processors are further programmed to: compare the output to a target leaf sequence corresponding to each fluence map; determine a difference between the leaf sequence and the target leaf sequence; and update one or more weights of the model based on the difference between the leaf sequence and the target leaf sequence.

20. The method of any one of clauses 16 to 19, wherein the one or more processors programmed to receive the set of fluence maps are programmed to: receive the set of fluence maps based on generation of treatment plans for patients involved in intensity-modulated radiation therapy (IMRT) or volumetric modulated arc therapy (VMAT) treatment.

21. The method of any one of clauses 16 to 19, wherein the one or more processors programmed to receive the set of fluence maps are programmed to: receive the set of fluence maps based on generation of a sample fluence map training set, the sample fluence map training set comprising one or more randomly-generated fluence maps.

22. The method of any one of clauses 16 to 19, wherein the one or more processors programmed to receive the set of fluence maps are programmed to: receive the set of fluence maps based on generation of a sample fluence map training set and generation of treatment plans for patients involved in intensity-modulated radiation therapy (IMRT) or volumetric modulated arc therapy (VMAT) treatment.

23. The method of any one of clauses 16 to 22, wherein the one or more processors that provide the data associated with each fluence map to the model to cause the model to generate the output are programmed to: provide the data associated with each fluence map to a generative transformer model to cause the generative transformer model to generate the output.

24. The method of any one of clauses 16 to 23, wherein the one or more processors that provide the data associated with each fluence map to the model to cause the model to generate the output are programmed to: provide the data associated with each fluence map to the model to cause the model to generate the output, the output representing a leaf sequence for the fluence map and at least one monitor unit (MU) value, the leaf sequence comprising one or more sets of multi-leaf collimator opening sequences corresponding to an MU value from among the at least one MU value.

25. A non-transitory computer-readable medium storing instructions thereon that, when executed by one or more processors, cause the one or more processors to: receive data associated with a set of fluence maps associated with operation of a multi-leaf collimator to focus energy generated by a linear accelerator (LINAC); and provide data associated with each fluence map to a model to cause the model to generate an output, the output representing a leaf sequence for the fluence map, the leaf sequence comprising one or more sets of multi-leaf collimator opening sequences.

26. The non-transitory computer-readable medium of clause 25, wherein the instructions that cause the one or more processors to provide the data associated with each fluence map to the model to cause the model to generate the output cause the one or more processors to: train the model to map input fluence maps to subsets of leaf sequences from among a set of possible leaf sequences.

27. A system, comprising: one or more processors programmed to: receive data associated with treatment attributes associated with a patient; generate, based on a first model trained to receive as input data associated with treatment attributes of patients, data associated with a fluence map for the patient; generate, based on a second model trained to receive fluence maps, data associated with a leaf sequence for the fluence map, the leaf sequence comprising one or more sets of multi-leaf collimator opening sequences; and transmit data associated with the leaf sequence to cause a linear accelerator (LINAC) to operate in accordance with the leaf sequence during delivery of energy to the patient.

By implementing models to both determine fluence maps and leaf sequences, the need for repetitive computation (e.g., with respect to inverse optimization or repetitive dose calculations and fluence map updates) the overall time needed to generate a treatment plan for a patient (e.g., going from data associated with the geometries of the patient, the planning target volume, and the organs at risk to a set of control points according to which a LINAC can be operated) can be reduced. This, in turn, can result in greater throughput of patients and higher utilization rates of LINACs, which in turn can lead to positive health outcomes as patients may be able to be treated faster than is possible with conventional techniques.

For the avoidance of doubt, the above-mentioned data associated with a set of fluence maps associated with operation of a multi-leaf collimator to focus energy generated by a linear accelerator (LINAC) can be data associated with a set of fluence maps generated by the model described herein, or maybe any other data associated with a set of fluence maps.

The preceding description of the disclosed embodiments is provided to enable any person skilled in the art to make or use the embodiments described herein and variations thereof. Various modifications to these embodiments will be readily apparent to those skilled in the art, and the principles defined herein can be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A system comprising:
   one or more processors programmed to:

   receive radiotherapy treatment data associated with a set of treatments administered to a set of patients that were previously treated, where each treatment of the set of treatments corresponds to a patient of the set of patients;
   generate a beam eye view, BEV, projection for each patient of the set of patients based on treatments of the set of treatments administered to each patient of the set of patients; and
   for each patient of the set of patients, provide treatment data associated with the patient and data associated with the BEV projections corresponding to the patient to a model (308) to train the model (308) to generate an output, the output representing a fluence map (304, 312).

2. The system of claim 1, wherein the one or more processors programmed to generate the BEV projection for each patient of the set of patients are programmed to:

   generate a set of digitally reconstructed radiographs, DRRs, based on the treatments of the set of treatments for each patient of the set of patients; and
   concatenate the set of DRRs for each patient to form the BEV projection for the patient.

3. The system of claim 2, wherein the one or more processors programmed to generate the set of DRRs are programmed to:
   generate the set of DRRs based on the treatments of the set of treatments for each patient of the set of patients and configurations of a multi-leaf collimator,

MLC, involved in the treatments of the set of treatments.

4. The system of any one of claims 1 to 3, wherein the one or more processors programmed to provide the treatment data associated with each patient and the data associated with the BEV projections associated with each patient to the model (308) are programmed to:
   provide the treatment data associated with the patient and the data associated with the BEV projections for each patient to the model (308), the treatment data associated with the patient comprising a representation of a planning target volume, PTV, or a representation of an organ at risk, OAR, for the patient.

5. The system of any one of claims 1 to 4, wherein the one or more processors programmed to provide the treatment data associated with each patient and the data associated with the BEV projections associated with each patient to the model (308) are programmed to:
   provide the treatment data associated with the patient and the data associated with the BEV projections for each patient to the model (308), the treatment data associated with the patient comprising one or more leaf configurations of a multi-leaf collimator, MLC, during transmission of energy by a linear accelerator, LINAC, to the patient.

6. The system of any one of claims 1 to 5, wherein the one or more processors programmed to provide the treatment data associated with each patient and the data associated with the BEV projections associated with each patient to the model (308) to train the model (308) to generate an output are programmed to:

   compare the output to a target fluence map to determine a difference between the output and the target fluence map; and
   update one or more weights of the model (308) based on the difference between the output and the target fluence map.

7. The system of any one of claims 1 to 6, wherein the one or more processors programmed to provide the treatment data associated with each patient and the data associated with the BEV projections associated with each patient to the model (308) to train the model (308) to generate an output are programmed to:

   provide the treatment data associated with each patient and the data associated with the BEV projections associated with each patient to the model (308) to train the model (308) to generate a first output, the first output associated with a first scale; and

provide the treatment data associated with each patient and the data associated with the BEV projections associated with each patient to the model (308) to train the model (308) to generate the output, the output associated with a second scale.

8. A method comprising:

receiving, by at least one processor, radiotherapy treatment data associated with a set of treatments administered to a set of patients that were previously treated, where each treatment of the set of treatments corresponds to a patient of the set of patients;

generating, by the at least one processor, a beam eye view, BEV, projection for each patient of the set of patients based on treatments of the set of treatments administered to each patient of the set of patients; and

for each patient of the set of patients, providing, by the at least one processor, treatment data associated with the patient and data associated with the BEV projections corresponding to the patient to a model (308) to train the model (308) to generate an output, the output representing a fluence map.

9. The method of claim 8, wherein generating the BEV projection for each patient of the set of patients comprises:

generating, by the at least one processor, a set of digitally reconstructed radiographs, DRRs, based on the treatments of the set of treatments for each patient of the set of patients; and

concatenating, by the at least one processor, the set of DRRs for each patient to form the BEV projection for the patient.

10. The method of claim 9, generating the set of DRRs comprises: generating, by the at least one processor, the set of DRRs based on the treatments of the set of treatments for each patient of the set of patients and configurations of a multi-leaf collimator, MLC, involved in the treatments of the set of treatments.

11. The method of any one of claims 8 to 10, wherein providing the treatment data associated with each patient and the data associated with the BEV projections associated with each patient to the model (308) comprises:

providing, by the at least one processor, the treatment data associated with the patient and the data associated with the BEV projections for each patient to the model (308), the treatment data associated with the patient comprising a representation of a planning target volume, PTV, or a representation of an organ at risk, OAR, for the patient.

12. The method of any one of claims 8 to 11, wherein providing the treatment data associated with each patient and the data associated with the BEV projections associated with each patient to the model (308) comprises:

providing, by the at least one processor, the treatment data associated with the patient and the data associated with the BEV projections for each patient to the model (308), the treatment data associated with the patient comprising one or more leaf configurations of a multi-leaf collimator, MLC, during transmission of energy by a linear accelerator, LINAC, to the patient.

13. The method of any one of claims 8 to 12, wherein providing the treatment data associated with each patient and the data associated with the BEV projections associated with each patient to the model (308) to train the model (308) to generate an output comprises:

comparing, by the at least one processor, the output to a target fluence map to determine a difference between the output and the target fluence map; and

updating, by the at least one processor, one or more weights of the model (308) based on the difference between the output and the target fluence map.

14. The method of any one of claims 8 to 13, wherein providing the treatment data associated with each patient and the data associated with the BEV projections associated with each patient to the model (308) to train the model (308) to generate an output comprises:

providing, by the at least one processor, the treatment data associated with the patient and the data associated with the BEV projections associated with the patient to the model (308) to train the model (308) to generate a first output, the first output associated with a first scale; and providing, by the at least one processor, the treatment data associated with the patient and the data associated with the BEV projections associated with the patient to the model (308) to train the model (308) to generate the output, the output associated with a second scale.

15. A non-transitory computer-readable medium storing instructions thereon that, when executed by one or more processors, cause the one or more processors to:

receive radiotherapy treatment data associated with a set of treatments administered to a set of patients that were previously treated, where each treatment of the set of treatments corresponds to a patient of the set of patients; generate a beam eye view (BEV) projection for each patient of the set of patients based on treatments of the set of treatments administered to each patient of the set of patients; and for each patient of the set of patients, provide treatment data associated with the patient and data associated with the BEV projections corresponding to the patient to a model (308) to train the model (308) to generate an output, the output representing a fluence map.

**FIG. 1**

EP 4 613 327 A1

200

Receive radiotherapy treatment data associated with a set of treatments administered to a set of patients that were previously treated. **202**

Generate a beam eye view (BEV) projection for each patient of the set of patients. **204**

For each patient of the set of patients, provide treatment data associated with the patient and data associated with the BEV projections corresponding to the patient to a model to train the model to generate an output, the output representing a fluence map. **206**

# FIG. 2

EP 4 613 327 A1

FIG. 3A

FIG. 3B

**FIG. 3C**

400

Receive data associated with a set of fluence maps for operation of a multi-leaf collimator to focus energy generated by a linear accelerator (LINAC). **402**

Provide data associated with each fluence map to a model to cause the model to generate an output, the output representing a leaf sequence for the fluence map, the leaf sequence comprising one or more sets of multi-leaf collimator opening sequences. **404**

Update one or more weights of the model based on a difference between the leaf sequence and target leaf sequence. **406**

# FIG. 4

EP 4 613 327 A1

FIG. 5A

FIG. 5B

EP 4 613 327 A1

**FIG. 5C**

EP 4 613 327 A1

Set of fluence maps

Set of (feasible) leaf sequences

x

Forward model

Non-realizable fluence maps

Realizable fluence maps

Equivalent optimal solutions

FIG. 5D

EP 4 613 327 A1

FIG. 5E

EP 4 613 327 A1

600

```
┌─────────────────────────────────────────────────────────────────────┐
│  Receive radiotherapy treatment data associated with the treatment    │
│  of a patient.                                                         │
│                              602                                      │
└─────────────────────────────────────────────────────────────────────┘
                                  │
                                  ▼
┌─────────────────────────────────────────────────────────────────────┐
│  Executing a first model to predict a fluence map for the patient     │
│  based on the radiotherapy treatment data. 604                        │
└─────────────────────────────────────────────────────────────────────┘
                                  │
                                  ▼
┌─────────────────────────────────────────────────────────────────────┐
│  Executing a second machine learning model to predict a leaf          │
│  sequence based on the fluence map. 606                               │
└─────────────────────────────────────────────────────────────────────┘
                                  │
                                  ▼
┌─────────────────────────────────────────────────────────────────────┐
│  Transmitting the leaf sequence to a plan optimizer computer model.   │
│  608                                                                  │
└─────────────────────────────────────────────────────────────────────┘
```

# FIG. 6

FIG. 7

EP 4 613 327 A1

## EUROPEAN SEARCH REPORT

**Application Number**

EP 25 16 1288

Europäisches Patentamt
European Patent Office
Office européen des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/254275 A1 (WU QINGRONG JACKIE [US] ET AL) 13 August 2020 (2020-08-13) * paragraphs [0003], [0027], [0030], [0032] - [0053]; claims 1-27; figures 1-4 * | 1-15 | INV. A61N5/10 G16H20/40 |
| A | US 2022/088410 A1 (HIBBARD LYNDON STANLEY [US]) 24 March 2022 (2022-03-24) * paragraphs [0001], [0008], [0047], [0048], [0065], [0099], [0120], [0181]; claims 11,12; figures 1-7 * | 1-15 | |
| A | US 2022/241614 A1 (WU QINGRONG [US] ET AL) 4 August 2022 (2022-08-04) * paragraphs [0007] - [0010], [0047], [0081] * | 1-15 | |
| A | US 2018/165423 A1 (KUUSELA ESA [FI] ET AL) 14 June 2018 (2018-06-14) * paragraphs [0024], [0081] * | 1-15 | |
| A | GOOD DAVID ET AL: "A Knowledge-Based Approach to Improving and Homogenizing Intensity Modulated Radiation Therapy Planning Quality Among Treatment Centers: An Example Application to Prostate Cancer Planning", INTERNATIONAL JOURNAL OF RADIATION: ONCOLOGY BIOLOGY PHYSICS, PERGAMON PRESS, USA, vol. 87, no. 1, 25 April 2013 (2013-04-25) , pages 176-181, XP028688871, ISSN: 0360-3016, DOI: 10.1016/J.IJROBP.2013.03.015 * the whole document * | 1-15 | TECHNICAL FIELDS SEARCHED (IPC) A61N G16H |

The present search report has been drawn up for all claims

| Place of search | Date of completion of the search | Examiner |
|---|---|---|
| Munich | 10 June 2025 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 16 1288

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2025

| Patent document cited in search report | Publication date | Patent family member(s) | Publication date |
|---|---|---|---|
| US 2020254275 A1 | 13-08-2020 | US 2020254275 A1<br>WO 2017087985 A1 | 13-08-2020<br>26-05-2017 |
| US 2022088410 A1 | 24-03-2022 | CN 116391234 A<br>EP 4200872 A1<br>US 2022088410 A1<br>WO 2022061324 A1 | 04-07-2023<br>28-06-2023<br>24-03-2022<br>24-03-2022 |
| US 2022241614 A1 | 04-08-2022 | NONE | |
| US 2018165423 A1 | 14-06-2018 | NONE | |

EPO FORM P0459

For more details about this annex : see Official Journal of the European Patent Office, No. 12/82